## Europäisches Patentamt

## European Patent Office

## Office européen des brevets

(19)

(11) Publication number: **0 249 418**
**A2**

(12) **EUROPEAN PATENT APPLICATION**

(21) Application number: **87305028.0**

(22) Date of filing: **08.06.87**

(51) Int. Cl.³: **G 01 N 33/543**

(30) Priority: **09.06.86 US 872358**

(43) Date of publication of application:
**16.12.87 Bulletin 87/51**

(84) Designated Contracting States:
**AT BE CH DE FR GB IT LI LU NL SE**

(71) Applicant: **ORTHO DIAGNOSTIC SYSTEMS INC.**
**One Johnson & Johnson Plaza**
**New Brunswick New Jersey 08933-7003(US)**

(72) Inventor: **Graham, Henry A., Jr.**
**RD2, Box 74 Sand Hill Road**
**Annandale New Jersey 08801(US)**

(74) Representative: **Jones, Alan John et al,**
**CARPMAELS & RANSFORD 43 Bloomsbury Square**
**London, WC1A 2RA(GB)**

(54) **Immunoglobulin immunoassays with an integrated control procedure.**

(57) There are provided new and improved methods for conducting immunoglobulin immunoassays which automatically includes the conduct of an integral control procedure wherein the successful conduct of the assay coupled with active components and the presence of the immunoglobulin to be detected by the immunoassay is necessary in order to obtain a positive result.

Croydon Printing Company Ltd.

METHODS FOR PERFORMING IMMUNOGLOBULIN IMMUNOASSAYS WITH
INTERNALIZED CONTROL AS AN INTEGRAL PART OF A POSITIVE
RESULT

Field of the Invention

This invention is related to the performance of
immunoassays for the detection of ligands and ligand
binding partners in bodily fluid samples and more
particularly, provides methods for controlling the
performance of immunoglobulin assays whereby a working
control is necessary for a positive result.

Background of the Invention

It is well-known that immunoassays are useful for
detecting analytes in patient fluid samples for the
purposes of diagnosing disease. Such analytes include for
instance antibodies which are proteins which react
specifically with antigens or haptens hereafter referred
interchangeably to as ligands and ligand binding
partners. Based on the specificity of such reactions, and
the ability to label components, .g. the biologically
active molecules, of such reactions with fluorochromes,
enzymes, light scattering particles and the like optically
detectable labels, one can determine with great
sensitivity the presence of ligands or ligand binding
partners in urine, blood, serum, cerebral spinal fluid,
solubilized body discharges and the like samples.
Correlation of the presence of these materials with
diseases assists greatly in the diagnosis and therpeutic
treatment of disease.

There is thus a continuing need for immunoassays of
greater sensitivity for detecting the early onset of

0249418

disease. To optimize practicality, however, such assays advantageously avoid dependence upon expensive instrumentation or other health related risks such as those associated with isotopic labels. Indeed, copending European patent application Nos.

and (claiming priority from USSNs 872 355 and 872 357 (ORD 63 and ORD 65) respectively) filed contemporaneously herewith, report upon two new immunoassay techniques both of which rely upon the use of colloidal gold as a label for producing visually detectable results.

Conventional assays often rely upon the ability of the technician to discriminate between a positive result and a negative result based on the presence or absence of an optically detectable material in a location. Often, such presence or absence is identifiable by a simple spot and while in most cases, such is perfectly adequate, in those instances when the biologically active molecule to be detected is present in low concentrations, only weakly positive reactions occur. Such weak positives are often difficult to detect or to objectively classify.

It is an object of the present invention to provide methods useful for colloidal gold immunoassays and other immunoassays generally for improving the identification of a positive result.

While improvements in manufacturing processes have resulted in tremendous reliability of the assays generally, including their sensitivity, specifically, virtually all assays can benefit from the performance of a control in order to ensure that the assay components are performing properly, that the operational aspects of the assay are being performed properly, and to provide a comparative result particularly useful for the inexperienced assay performer or where quantitative indications are useful.

It is another aspect of the present invention to provide new methods for incorporating control procedures within an immunoassay system.

It is yet another aspect of the present invention to provide methods for performing a control procedure at the same time as the assay is performed.

It is yet still another aspect of the present invention to combine the performance of controls with an improvement in visual detectability of positive results.

Conventional controls for assay procedures do not ensure that sample additions have taken place, a problem often encountered in the clinical environment where interruptions are the rule rather than the exception.

It is still another aspect of the present invention to provide control methods especially useful for immunoglobulin assays for ensuring that assay components are active and assay procedures including sample addition and washing steps are performed properly.

## Summary of the Invention

In accordance with the principles and aspects of the present invention, there are provided new control procedures which form an integral part of the performance of an immunoassay such that the proper performance of the procedure coupled with active immunoassay ingredients in conjunction with a positive sample is necessary in order to achieve an observable positive reaction. Further, to improve the detectability of such a positive result, the control and sample results are ideally combined in a pattern to assist the operator in discriminating a positive result from a negative or no result condition. The invention is especially useful for ensuring that sample has been properly added and washed in immunoglobulin assays.

In the most preferred embodiment, a "plus" pattern is employed wherein one leg of the plus results from successful control while the other leg of the plus results from the presence of the ligand or ligand binding partner to be detected in the sample. Failure of the reagents or the operator to perform the assay in a correct manner results in a failure of the control portion or zone of the readout and hence a "plus" readout does not appear and a false positive is avoided. Similarly, if the assay components are active and the assay is performed properly, a "minus" readout will result in the absence of ligand or ligand binding partner in the sample. Only if the reagent materials are active, the assay is performed properly, and the ligand or ligand binding partner is present in the sample, will a "plus" obtain due to 'labeling' of both the control and test zones.

The present invention is particularly useful with respect to immunoglobulin based assays in that a readout from the control portion or zone of the assay will only occur if the sample has been added. This is particularly important in such tests when the clinician is constantly interrupted during the conduct of multiple procedures and hence becomes unsure during such interruptions whether patient samples have been added to a particular test site or not. In such a preferred test, the test zone has ligand attached thereto and specific for the immunoglobulin to be detected in the aqueous human sample. The control zone has anti-human IgG or IgM immobilized thereon for nonspecifically capturing human immunoglobulin ubiquitously present in all similar human aqueous samples. The performance of a wash step removes unbound materials and thus sites for labeling prior to contact with the labeled anti-human IgG or IgM.

Detailed Description of the Invention and Best Mode

While the instant invention may be utilized with a variety of different immunoassay procedures employing different detectable labels, for ease of explanation, the colloidal gold assays of the copending European patent applications, previously mentioned, will be used to describe the invention by way of example. It will be understood, however, that the invention is not limited to such assays or labels and those skilled in the art will readily be able to determine the appropriate substitutions with regard to immunoassays employing different labels or surfaces.

The COGMA assays of (ORD-63) benefit greatly from one aspect of the instant invention in that a "plus" or a "minus" is more readily identified than a "spot" which by nature is less clearly defined. Using the detection of human immunoglobulin specific for Rubella (an indicator of exposure to measles) as a suitable example, one would employ a membrane having biologically active components or molecules arrayed such as the following:

```
                        R
                        U
                        B
                        E
                        L
                        L
                        A
      M O U S E   A N T I - H U M A N   A N T I B O D Y
                        A
                        N
                        T
                        I
                        G
                        E
                        N
```

A whole blood sample or blood component (e.g. serum or plasma) containing immunoglobulin of many different specificities and may contain immunoglobulin specific for Rubella. The addition of such a sample to the membrane permits capture of immunoglobulin nonspecifically by the mouse (or any species) anti-human antibody. If Rubella specific immunoglobulin is also present, it will be captured by the vertical zone. The Rubella antigen may have been attached to the membrane directly or through a chemical linker or by immunological complexing. A wash step is then performed in order to displace unattached immunoglobulins from the surface which would otherwise result in non-localized attachment of labeled reagent.

Subsequently, colloidal gold labeled anti-human IgG or IgM is contacted with the membrane and it reacts with the immunoglobulin from the sample now immobilized on the horizontal strip, as well as any Rubella specific immunoglobulin present in the sample and captured in the vertical zone. Thus, a "plus" results if there is presence of the Rubella immunoglobulin in the sample. Absence of Rubella immunoglobulin in the urine would result in no attachment of immunoglobulin to the vertical line and thus only attachment of the colloidal gold labeled anti-human antibody to the horizontal control zone containing the anti-human immunoglobulin which, like the labeled immunoglobulin, may be either IgG or IgM and of either polyclonal or monoclonal origin. It will be further perceived that other patterns may be equally employed including circles with lines through them, squares, triangles, actual words and the like. From a manufacturing perspective and a simplicity of operation particularly for the generally public, a "plus" and a "minus" pattern is most preferred. Other substitutions may be readily apparent and include for instance the direct application of antigen or ligand itself to the membrane (generally more difficult to attach than an antibody-ligand complex) as well as the substitution of different types of labels or immunoglobulins from different species. Similarly, the aforementioned control will work equally well, albeit with obvious substitutions, for other immunoglobulin tests, labeling methods or surfaces.

For example, the detection of immunoglobulins in the presence of body fluids such as urine, blood serum, plasma, etc. important in the detection of Rubella, toxoplasmosis, cytomeglavirus, HTLV-III (for AIDS), mononucleosis, Herpes, hepatitis and the like diseases, one places the ligand or equivalent immunologically reactive component for which the antibodies are specific on the membrane in order to capture those antibodies. The labeled component may then be suitably and ideally selected as an anti-human IgG or IgM as appropriate while the control portion of the membrane may also be suitably selected to be an anti-human IgG or IgM for capturing the labeled component. Alternately, one may put onto the control zone any biologically active molecule which binds a component in the sample which is known to be present in all such samples. The labeled reagent may then contain a similar labeled biologically active molecule and/or a labeled anti-human immunoglobulin which can react with both the sample component and the ligand specific, sample immunoglobulin.

Thus, in the presence of the antibody to be detected, a "plus" results while the absence of that antibody results in a "negative". Such a negative indicates that the membrane anti-human antibody and the labeled anti-human immunoglobulin were both working. It further indicates that the sample was properly added (otherwise labeled anti-human immunoglobulin would not be localized), and that the post sample addition, wash step was performed (otherwise a "spot" rather than a "minus" would be visible) and that these steps were performed in the appropriate order. Absence of a "minus" therefore indicates failure of the test.

The instant invention is also useful with the strip element assays described in *U.S.S. N.* (ORD-65).

In such assays a first or lower band, applied crosswise to the longitudinal axis of the strip element, has attached therein Rubella or other antigen thereby forming the test zone for detection of Rubella specific immunoglobulin in a blood sample. An upper band, located more distant to the end of the strip element brought in contact with the sample, has attached thereto anti-human immunoglobulin serving the a control zone. The blood sample is then

-66

applied to the end of the strip element. Presence of Rubella immunoglobulin results in its attachment at the lower band while other sample immunoglobulin is nonspecifically attached to the upper band. Colloidal gold labeled anti-human antibody (IgG or IgM) is contacted with the bottom of the strip and allowed to migrate after unreacted components have been washed away. The upper control band thereby serves to show that the strip element components and the colloidal gold labeled component was active and properly applied to the strip element. The same component substitution possibilities described in the first example also pertain to such strip assays. Further, such strip assays may provide multiple test zones and possibly also multiple control zones in order to make the strip applicable to testing many conditions (e.g. TORCH) or useful for different sample types/ligand or ligand binding partner tests.

Thus, the control concepts of the present invention provide significant advantages for immunoglobulin tests. All steps of the immunoassay are controlled in that the human serum in the sample addition reacts with the membrane bound (mouse monoclonal) anti-human IgG (or IgM). If the sample is not added, the human IgG (or IgM) would not bind to the membrane bound anti-IgG region. It further ensures that the wash is performed in an adequate manner. Failure to adequately wash results in free human IgG in or on the surface neutralizing the labeled anti-human immunoglobulin reagent and thus the reaction between the labeled antibody and the membrane bound antibody region would be lessened or not appear. Further, if the labeled component is not added, no signal at all will be present or if added and not adequately removed such as by a washing step, no discernible "plus" or "minus" will be apparent. As a result, a true control,

not known previously, obtains for ensuring active components, sample addition, addequate wash steps, and labeled component addition.

It will be readily appreciated by those skilled in the art that numerous alterations to the foregoing may be made without departing from either the spirit or scope of the present invention.

CLAIMS

1. In an immunoassay for the detection of an immunoglobulin in a sample utilizing one or more ligand binding partners specific thereto, at least one of said ligand binding partners having a detectable label attached for determining when a reaction between the immunoglobulin and ligand binding partner has taken place, the improvement comprising providing an immunoassay control to be performed at the time and as part of the immunoassay wherein said control confirms the procedural steps of the assay including the performance of sample addition to the ligand binding partners and the performance of requisite wash steps.

2. The method as provided in Claim 1 wherein the control portion of the readout and the test portion of the readout together combine to form a discernible pattern.

3. The method as provided in Claim 2 wherein such pattern is a "plus" and wherein the control portion is one leg of the "plus" while the test portion is the other leg of the "plus".

4. An immunoassay for the detection of immunoglobulin in a human fluid sample comprising the steps of:

a) providing a first zone having a ligand for which the immunoglobuin is specific and a second zone having a binding substance capable of reacting with a component in the sample that is ubiquitously present in all human fluid samples undergoing similar immunoassay testing;

0249418

b) contacting said first and second zones with the human fluid sample;

c) removing unbound materials from said first and second zones;

d) contacting said first and second zones with a labeling reagent comprising labeled anti-human IgG or IgM capable of reacting with both said sample immunoglobulin and said sample component;

e) removing unreacted materials from said first and second zones; and

f) observing said first and second zones for the presence of label wherein the presence of label in both of said first and second zones is necessary in order to obtain a positive result.

5. The method as provided in Claim 4 wherein said first and second zones combine to form an identifiable pattern and wherein the presence of label in either of said first or second zones alone constitutes a negative result and the absence of label in both said first and second zones constitutes a failed immunoassay and the presence of label in said zones without forming an identifiable pattern constitutes a failed immunoassay.

6. The method as provided in Claim 4 or claim 5 wherein the label is selected from the group consisting of colloidal gold, fluorescent molecules, and enzymes.

7. The method of any one of claims 4 to 6 wherein said sample component is human immunoglobulin and said binding substance is anti-human immunoglobulin.

8.   The method of any one of claims 4 to 6 wherein said sample component is a protein other than immunoglobulin and said labelled reagent further comprises a labelled protein binding partner.

9.   In an immunoassay for the detection of an immunoglobulin in a sample utilizing one or more ligand binding partners specific thereto, at least one of said ligand binding partners having a detectable label attached for determining when a reaction between the immunoglobulin and ligand binding partner has taken place, the improvement comprising a zone capable of reacting with a sample component to ensure the sample was added, said sample component further reacting with said labeled reagent.